# EUROPEAN PATENT SPECIFICATION

(11) **EP 1 915 150 B1**
(45) Date of publication and mention of the grant of the patent: **26.01.2011**
(21) Application number: 06762350.4
(22) Date of filing: 03.07.2006
(51) Int. Cl.: A61K 31/4436, C07D 495/04, A61P 3/10

(54) **USE OF THIENOPYRIDONE DERIVATIVES AS AMPK ACTIVATORS AND PHARMACEUTICAL COMPOSITIONS CONTAINING THEM**
VERWENDUNG VON THIENOPYRIDONDERIVATEN ALS AMPK-AKTIVATOREN UND PHARMAZEUTISCHE ZUSAMMENSETZUNGEN, DIE DIESE ENTHALTEN
UTILISATION DE DÉRIVÉS DE THIÉNOPYRIDONE COMME ACTIVATEURS AMPK ET COMPOSITIONS PHARMACEUTIQUE QUI LES CONTIENNENT

(30) Priority: 18.08.2005 EP 05291753
(43) Date of publication of application: 30.04.2008
(73) Proprietor: Merck Patent GmbH, 64293 Darmstadt (DE)
(72) Inventor: HALLAKOU-BOZEC, Sophie, F-92120 Montrouge (FR); CHARON, Christine, F-91940 Gometz-le-Chatel (FR); HOCK, Bjoern, 63477 Maintal (DE); POESCHKE, Oliver, 65201 Wiesbaden (DE)
(86) International application number: PCT/EP2006/006446
(87) International publication number: WO 2007/019914

(56) References cited:
- US-A- 5 602 144
- US-A1- 2005 038 068
- BUCHSTALLER, H.-P.; SIEBERT, C. D.; LYSSY, R. H.; ECKER, G.; KRUG, M.; BERGER, M. L.; GOTTSCHLICH, R.; NOE, C. R.: "Thieno[2,3-b]pyridinones as Antagonists on the Glycine Site of the N-methyl-D-aspartate Receptor-Binding Studies, Molecular Modeling and Structure-Activity-Relationships" SCIENTIA PHARMAZEUTICA, vol. 68, 2000, pages 3-14, XP008059055

## Description

### FIELD OF THE INVENTION

This invention relates to thienopyridone derivatives that are activators of AMP-activated protein kinase (AMPK). The invention also relates to the preparation and use of these thienopyridones in the treatment of disorders such as diabetes, metabolic syndrome and obesity.

### BACKGROUND AND INTRODUCTION TO THE INVENTION

AMPK is well established as a sensor and regulator of cellular energy homeostasis (Hardie, D. G. and Hawley, S. A., "AMP- activated protein kinase: the energy charge hypothesis revisited", Bioassays, 23, 1112, (2001), Kemp, B. E. et al., "AMP-activated protein kinase, super metabolic regulator', Biochem. Soc. Transactions, 31, 162 (2003)). Allosteric activation of this kinase due to rising AMP levels occurs in states of cellular energy depletion. The resulting serine/threonine phosphorylation of target enzymes leads to an adaptation of cellular metabolism to the low energy state. The net effect of AMPK activation induced changes is inhibition of ATP consuming processes and activation of ATP generating pathways, and therefore regeneration of ATP stores. Examples of AMPK substrates include acetyl-CoA-carboxylase (ACC) and HMG-CoA-reductase (Carling D. et al., "A common bicyclic protein kinase cascade inactivates the regulatory enzymes of fatty acid and cholesterol biosynthesis", FEBS Letters, 223, 217 (1987)). Phosphorylation and therefore inhibition of ACC leads to a decrease in fatty acid synthesis (ATP-consuming) and at the same time to an increase in fatty acid oxidation (ATP-generating). Phosphorylation and resulting inhibition of HMG-CoA reductase leads to a decrease in cholesterol synthesis. Other substrates of AMPK include hormone sensitive lipase (Garton, A. J. et al., "Phosphorylation of bovine hormone-sensitive lipase by the AMP-activated protein kinase. A possible antilipolytic mechanism", Eur. J. Biochem., 179, 249, (1989)), glycerol-3- phosphate acyltransferase (Muoio D. M. et al., "AMP-activated kinase reciprocally regulates triacylglycerol synthesis and fatty acid oxidation in liver and muscle: evidence that sn-glycerol-3-phosphate acyltransferase is a novel target", Biochem. J., 338, 783, (1999)), malonyl-CoA decarboxylase (Saha A. K. et al., "Activation of malonyl-CoA decarboxylase in rat skeletal muscle by contraction and the AMP-activated protein kinase activator 5-aminoimidazole-4-carboxamide-1-β-D-ribofura-noside", J. Biol. Chem., 275, 24279, (2000)), and hepatocyte nuclear factor-4α (Leclerc I. et al., "Hepatocyte nuclear factor-4α involved in type-1 maturity-onset diabetes of the young is a novel target of AMP-activated protein kinase", Diabetes, 50, 1515, (2001)), some of which are potential drug targets for components of the metabolic syndrome. Additional processes that are believed to be regulated through AMPK activation, but for which the exact AMPK substrates have not been identified, include stimulation of glucose transport in skeletal muscle and expressional regulation of key genes in fatty acid and glucose metabolism in liver (Hardie D. G. and Hawley S. A., "AMP-activated protein kinase: the energy charge hypothesis revisited", Bioassays, 23, 1112, (2001), Kemp B. E. et al., "AMP-activated protein kinase, super metabolic regulator", Biochem. Soc. Transactions, 31, 162, (2003), Musi N. and Goodyear L. J., "Targeting the AMP-activated protein kinase for the treatment of type 2 diabetes", Current Drug Targets-Immune, Endocrine and Metabolic Disorders, 2, 119, (2002)). For example, decreased expression of glucose-6-phosphatase (Lochhead P. A. et al., "5-aminoimidazole-4-carboxamide riboside mimics the effects of insulin on the expression of the 2 key gluconeogenic genes PEPCK and glucose-6-phosphatase", Diabetes, 49, 896, (2000)), a key enzyme in hepatic glucose production, and SREBP-1c (Zhou G. et al., "Role of AMP-activated protein kinase in mechanism of metformin action", The J. of Clin. Invest., 108, 1167, (2001)), a key lipogenic transcription factor, has been found following AMPK stimulation.

More recently an involvement of AMPK in the regulation of not only cellular but also whole body energy metabolism has become apparent. It was shown that the adipocyte-derived hormone leptin leads to a stimulation of AMPK and therefore to an increase in fatty acid oxidation in skeletal muscle (Minokoshi Y. et al., "Leptin stimulates fatty-acid oxidation by activating AMP-activated protein kinase", Nature, 415, 339, (2002)). Adiponectin, another adipocyte derived hormone leading to improved carbohydrate and lipid metabolism, has been demonstrated to stimulate AMPK in liver and skeletal muscle (Yamauchi T. et al., "Adiponectin stimulates glucose utilization and fatty acid oxidation by activating AMP-activated protein kinase", Nature Medicine, 8, 1288, (2002), Tomas E. et al., "Enhanced muscle fat oxidation and glucose transport by ACRP30 globular domain: Acetyl-CoA carboxylase inhibition and AMP- activated protein kinase activation", PNAS, 99, 16309,(2002)). The activation of AMPK in these circumstances seems to be independent of increasing cellular AMP levels but rather due to phosphorylation by one or more yet to be identified upstream kinases.

Based on the knowledge of the above-mentioned consequences of AMPK activation, profound beneficial effects would be expected from in vivo activation of AMPK. In liver, decreased expression of gluconeogenic enzymes would reduce hepatic glucose output and improve overall glucose homeostasis, and both direct inhibition and/or reduced expression of key enzymes in lipid metabolism would lead to decreased fatty acid and cholesterol synthesis and increased fatty acid oxidation. Stimulation of AMPK in skeletal muscle would increase glucose uptake and fatty acid oxidation with resulting improvement of glucose homeostasis and, due to a reduction in intra-myocyte triglyceride accumulation, to improved insulin action. Finally, the increase in energy expenditure should lead to a decrease in body weight. The combination of these effects in the metabolic syndrome would be expected to significantly reduce the risk for acquiring cardiovascular diseases.

Several studies in rodents support this hypothesis (Bergeron R. et al., "Effect of 5-aminoimidazole-4-carboxamide-1(beta)-D-ribofuranoside infusion on in vivo glucose metabolism in lean and obese Zucker rats", Diabetes, 50, 1076, (2001), Song S. M. et a/., "5-Aminoimidazole- 4- dicarboxamide ribonucleoside treatment improves glucose homeostasis in insulin-resistant diabeted (ob/ob) mice", Diabetologia, 45, 56, (2002), Halseth A. E. et al., "Acute and chronic treatment of ob/ob and db/db mice with AICAR decreases blood glucose concentrations", Biochem. and Biophys. Res. Comm., 294, 798, (2002), Buhl E. S. et al., "Long-term AICAR administration reduces metabolic disturbances and lowers blood pressure in rats displaying feature of the insulin resistance syndrome", Diabetes, 51, 2199, (2002)). Until recently most in vivo studies have relied on the AMPK activator AICAR, a cell permeable precursor of ZMP. ZMP acts as an intracellular AMP mimic, and, when accumulated to high enough levels, is able to stimulate AMPK activity (Corton J. M. et al., "5-Aminoimidazole-4- carboxamide ribonucleoside, a specific method for activating AMP- activated protein kinase in intact cells?", Eur. J. Biochem., 229, 558, (1995)). However, ZMP also acts as an AMP mimic in the regulation of other enzymes, and is therefore not a specific AMPK activator (Musi N. and Goodyear L. J., "Targeting the AMP-activated protein kinase for the treatment of type 2 diabetes", Current Drug Targets-Immune, Endocrine and Metabolic Disorders, 2, 119 (2002)). Several *in vivo* studies have demonstrated beneficial effects of both acute and chronic AICAR administration in rodent models of obesity and type 2 diabetes (Bergeron R. et al., "Effect of 5-aminoimidazole-4-carboxamide-1β-D-ribofuranoside infusion on in vivo glucose metabolism in lean and obese Zucker rats", Diabetes, 50,1076, (2001), Song S. M. et al., "5-Aminoimidazole- 4-darboxamide ribonucleoside treatment improves glucose homeostasis in insulin-resistant diabeted (ob/ob) mice", Diabetologia, 45, 56, (2002), Halseth A. E. et al., "Acute and chronic treatment of ob/ob and db/db mice with AICAR decreases blood glucose concentrations", Biochem. and Biophys. Res. Comm., 294, 798, (2002), Buhl E. S. et al., "Long-term AICAR administration reduces metabolic disturbances and lowers blood pressure in rats displaying feature of the insulin resistance syndrome", Diabetes, 51, 2199, (2002)). For example, 7 week AICAR administration in the obese Zucker (fa/fa) rat leads to a reduction in plasma triglycerides and free fatty acids, an increase in HDL cholesterol, and a normalization of glucose metabolism as assessed by an oral glucose tolerance test (Minokoshi Y. et al., "Leptin stimulates fatty-acid oxidation by activating AMP-activated protein kinase", Nature, 415, 339, (2002)). In both ob/ob and db/db mice, 8 day AICAR administration reduces blood glucose by 35% (Halseth A. E. et al., "Acute and chronic treatment of ob/ob and db/db mice with AICAR decreases blood glucose concentrations", Biochem. and Biophys. Res. Comm., 294, 798, (2002)). In addition to AICAR, more recently it was found that the diabetes drug metformin can activate AMPK *in vivo* at high concentrations (Zhou G. et al., "Role of AMP-activated protein kinase in mechanism of metformin action", The J. of Clin. Invest, 108, 1167, (2001), Musi N. et al., "Metformin increases AMP-activated protein kinase activity in skeletal muscle of subjects with type 2 diabetes", Diabetes, 51, 2074, (2002)), although it has to be determined to what extent its antidiabetic action relies on this activation. As with leptin and adiponectin, the stimulatory effect of metformin is indirect via activation of an upstream kinase (Zhou G. et al., "Role of AMP-activated protein kinase in mechanism of metformin action", The J. of Clin. Invest., 108, 1167, (2001)). In addition to pharmacologic intervention, several transgenic mouse models have been developed in the last years, and initial results are becoming available. Expression of dominant negative AMPK in skeletal muscle of transgenic mice has demonstrated that the AICAR effect on stimulation of glucose transport is dependent on AMPK activation (Mu J. et al., "A role for AMP-activated protein kinase in contraction and hypoxia-regulated glucose transport in skeletal muscle", Molecular Cell, 7, 1085, (2001)), and therefore likely not caused by non- specific ZMP effects. Similar studies in other tissues will help to further define the consequences of AMPK activation. It is expected that pharmacologic activation of AMPK will have benefits in the metabolic syndrome with improved glucose and lipid metabolism and a reduction in body weight. To qualify a patient as having metabolic syndrome, three out of the five following criteria must be met: elevated blood pressure above 130/85 mmHg, fasting blood glucose above 110 mg/dl, abdominal obesity above 40" (men) or 35" (women) waist circumference, and blood lipid changes as defined by an increase in triglycerides above 150 mg/dl or decreased HDL cholesterol below 40 mg/dl (men) or 50 mg/dl (women). Therefore, the combined effects that may be achieved through activation of AMPK in a patient who qualifies as having metabolic syndrome would raise the interest of this target.

Stimulation of AMPK has been shown to stimulate expression of uncoupling protein 3 (UCP3) in skeletal muscle (Zhou M. et al., "UCP-3 expression in skeletal muscle: effects of exercise, hypoxia, and AMP-activated protein kinase", Am. J. Physiol. Endocrinol. Metab., 279, E622, (2000)) and might therefore be a way to prevent damage from reactive oxygen species. Endothelial NO synthase (eNOS) has been shown to be activated through AMPK mediated phosphorylation (Chen Z.-P., et al., "AMP-activated protein kinase phosphorylation of endothelial NO synthase", FEBS Letters, 443, 285, (1999)), therefore AMPK activation can be used to improve local circulatory systems.

In addition to AICAR and derivatives, other AMPK activators have been described (WO 2005/928464, US 2005/038068, WO 2004/043957).

### DESCRIPTION OF THE INVENTION

US 5602144 describes and claims new thienopyridone derivatives as compounds useful for the treatment of neurodegenerative diseases. It has now been discovered that these compounds and some new analogues are activator of AMP-activated protein kinase (AMPK) and therefore useful for the treatment of disorders such as diabetes, metabolic syndrome and obesity. The invention relates to the use of the compounds described in US 5602144, incorporated herein, and of certain new thienopyridone derivatives, said known and new compounds being of formula (I): 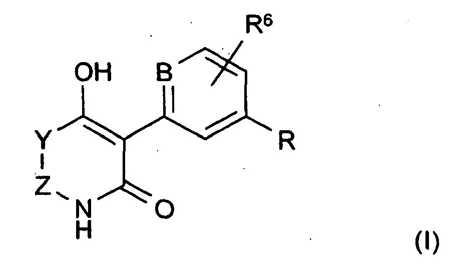 in which
B is CH or N, 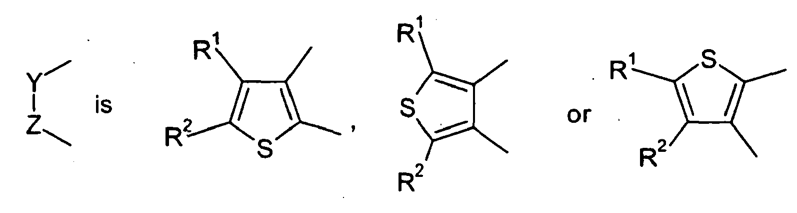 R is H, 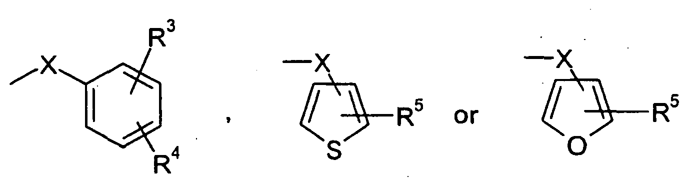 R¹ and R², independently from one another, represent H, linear or branched (C₁-C₄)alkyl, (C₁-C₄)cycloalkyl, (C₁-C₄)cycloalkyl(C₁-C₄)alkyl, halogen or,
together form a group -(CH₂)ₙ-, with n = 1 to 4,
R³ and R⁴, independently from one another, represent H, linear or branched (C₁-C₄)alkyl, OH, linear or branched (C₁-C₄)alkoxy, halogen, CF₃, NO₂, NH₂, NHR' where R' is linear or branched (C₁-C₆)acyl, NHR", N(R")₂, where R" is linear or branched (C₁-C₄)alkyl, optionally substituted anilino, optionally substituted phenoxy, optionally substituted benzyl, or optionally substituted benzoyl,
R⁵ is H, linear or branched (C₁-C₄)alkyl, or halogen,
R⁶ is H or halogen, linear or branched (C₁-C₄)alkyl, (C₁-C₄)cycloalkyl, (C₁-C₄)cycloalkyl(C₁-C₄)alkyl,
X is -CH₂-, -CO-, -O-, -S-, -NH-, or -NR"'- , where R"' is linear or branched (C₁-C₄)alkyl,
and their pharmaceutically acceptable salts,
for the preparation of a pharmaceutical composition useful for the treatment of diabetes, metabolic syndrome, related disorders and obesity.

Since these compounds may have asymmetric centers, the present invention is directed not only to racemic mixtures of these compounds, but also to individual stereoisomers and diastereoisomers, including their mixtures in all proportions. The present invention also includes pharmaceutically acceptable and/or useful salts of the compounds of formula (I), including acid addition salts. The present invention also includes oxides, and solvates, as well as eventual tautomeric forms. The present invention also encompasses prodrugs of compounds of formula (I).

The acids used for the preparation of the pharmaceutically acceptable salts are inorganic or organic acids. The resulting salts are for example hydrochlorides, hydrobromides, sulfates, hydrogenosulfates, dihydrogeno-phosphates, citrates, maleates, fumarates, trifluoroacetates, 2-naphtalene-sulfonates, *para*-toluenesulfonates.

The invention also relates to pharmaceutically acceptable salts with organic or inorganic bases. The resulting salts are for example sodium salts, potassium salts, lithium salts.

The invention also relates to the salts used for the chiral resolution of the racemates.

As examples the following chiral acids can be used: (+)-D-di-O-benzoyltartaric acid, (-)-L-di-O-benzoyltartaric acid, (-)-L-di-O,O'-p-toluyl-L-tartaric acid, (+)-D-di-O,O'-p-toluyl- L-tartaric acid, (*R*)-(+)-malic acid, (*S*)-(-)-malic acid, (+)-camphoric acid, (-)-camphoric acid, *R*-(-)-1,1'-binaphtalen-2,2'-diyl hydrogenophosphonic, (+)-camphanic acid, (-)-camphanic acid, (*S*)-(+)-2-phenylpropionic acid, (*R*)-(+)-2-phenylpropionic acid, D-(-)-mandelic acid, L-(+)-mandelic acid, D-tartaric acid, L-tartaric acid, or a mixture of them.

The term "prodrug" as used herein refers to any compound that when administered to a biological system generates the "drug" substance (a biologically active compound) as a result of spontaneous chemical reaction(s), enzyme catalyzed chemical reaction(s), and/or metabolic chemical reaction(s). Standard prodrugs are formed using groups attached to functionality, *e.g*. HO-, HS-, HOOC- , R₂N-, associated with the AMPK activator, that cleave *in vivo.* Standard prodrugs include but are not limited to carboxylate esters where the group is alkyl, aryl, aralkyl, acyloxyalkyl, alkoxycarbonyloxyalkyl as well as esters of hydroxyl, thiol and amines where the group attached is an acyl group, an alkoxycarbonyl, aminocarbonyl, phosphate or sulfate. Prodrugs must undergo some form of a chemical transformation to produce the compound that is biologically active or is a precursor of the biologically active compound. In some cases, the prodrug is biologically active, usually less than the drug itself, and serves to imprive efficacy or safety through improved oral bioavailability, pharmacodynamic half-life, etc.

In accordance with the present invention and as used herein, the following terms are defined with the following meanings, unless explicitly stated otherwise.

The term "linear or branched (C₁-C₄)alkyl" refers to saturated aliphatic groups including straight chain or branched chain groups. Alkyl groups may be optionally substituted. Suitable groups include methyl, ethyl, propyl, butyl, isopropyl, isobutyl, ter-butyl, sec-butyl.

The term "linear or branched (C₁-C₄)cycloalkyl" refers to cyclopropyl or cyclobutyl.

The term "linear or branched (C₁-C₆)acyl" refers to saturated aliphatic alkyl-C(=O)- groups including straight chain or branched chain groups. Alkyl groups may be optionally substituted. Suitable (C₁-C₆)acyl groups include acetyl, propanoyl, butanoyl, pentanoyl, hexanoyl, isobutyryl.

The term "halogen" refers to Cl, Br, I, F.

The term "optionally substituted" or "substituted" includes groups substituted by one to four substituents, independently selected from lower alkyl, lower aryl, lower aralkyl, lower alicyclic, hydroxy, lower alkoxy, lower aryloxy, perhaloalkoxy, aralkoxy, heteroaryl, heteroaryloxy, heteroarylalkyl, heteroaralkoxy, azido, amino, guanidino, amidino. halo, lower alkylthio, oxo, acylalkyl, carboxy esters, carboxyl, -carboxamido, nitro, acyloxy, aminoalkyl, alkylaminoaryl, alkylaryl, alkylaminoalkyl, alkoxyaryl, arylamino, aralkylamino, phosphono, sulfonyl, -carboxamidoalkylaryl, -carboxamidoaryl, hydroxyalkyl, haloalkyl, alkylaminoalkylcarboxy-, aminocarboxamidoalkyl-, cyano, lower alkoxyalkyl, lower perhaloalkyl, perhaloalkoxy, and arylalkyloxyalkyl.

The underlying object of the invention was to discover novel compounds having valuable properties, in particular those which can be used to prepare medicaments.

According to one embodiment of the invention, it has been found that the compounds of the formula (I), and their physiologically harmless salts, possess valuable pharmacological properties. They are able to activate AMPK and are therefore suitable, for treating disorders regulated by the activation of AMPK.

The compounds are particularly suitable for the treatment or prophylaxis of diabetes, metabolic syndrome, obesity.

The compounds can therefore be used as medicinal active compounds in human and veterinary medicine. In addition, they are suitable as intermediates for preparing other compounds possessing valuable properties.

As already mentioned, compounds of formula (I) encompass compounds known from US 5,602,144, as well as new compounds.

The present invention therefore also pertains to such new compounds of formulae (I_{A}), and (I_{C}), as defined below, all compounds disclosed in US 5,602,144, together with compounds of formulae (I_{A}), and (I_{C}), forming the entire set of compounds of formula (I) as defined above.

Therefore, the present invention further relates to compounds of formula (I_{A}): 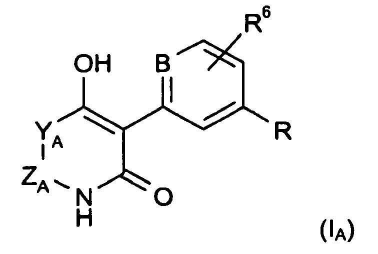 in which 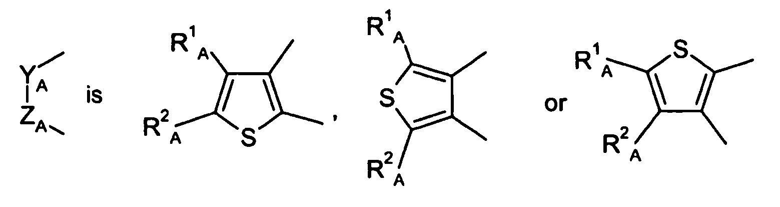 wherein R¹_{A} and R²_{A}, independently from one another, represent (C₁-C₄)cycloalkyl or (C₁-C₄)cycloalkyl(C₁-C₄)alkyl or,
together form a group -(CH₂)ₙ-, with n = 1 to 4,
B, R, R³, R⁴, R⁵, R⁶ and X being as defined above in formula (I),
and their pharmaceutically acceptable salts.

As new compounds, the present invention also refers to compounds of formula (I_{C}): 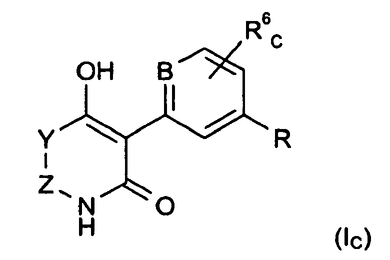 in which
R⁶ is linear or branched (C₁-C₄)alkyl, (C₁-C₄)cycloalkyl, or (C₁-C₄)cycloalkyl(C₁-C₄)alkyl,
B, -Y-Z-, R, R¹ R², R³, R⁴, R⁵ and X being as defined above in formula (I),
and their pharmaceutically acceptable salts excluding 4-hydroxy-5-(2-methylphenyl)-thieno [2,3-b] pyridine-6(7H)-one.

By "pharmaceutically acceptable salts" is meant the various salts obtained by reaction of compounds (I_{A}) to (I_{C}) with acids or bases as previously disclosed for compounds of formula (I). These salts also include salts useful for the chiral resolution of the racemates.

The present invention also encompasses the isomers of compounds (I_{A}) to (I_{C}) as defined above. The term "isomer" is as described above for compounds of formula (I) and comprises not only racemic mixtures of these compounds, but also individual stereoisomers and/or diastereoisomers, including their mixtures in all proportions.

Also encompassed are the oxides and solvates, as well as eventual tautomeric forms of compounds (I_{A}) to (I_{C}), and their corresponding prodrugs.

The invention relates to the use of compounds of formula (I) and their salts as defined above, and also to a process for preparing these compounds, and their salts, said process comprising the steps wherein:
1) a compound of formula (II) 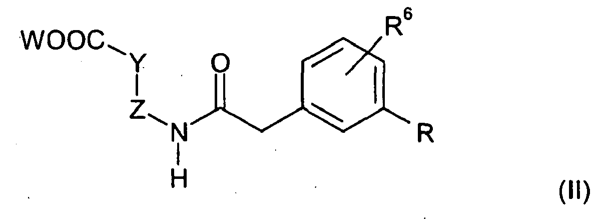 in which
   B, R, R⁶ and -Y-Z- have the above-mentioned meanings, and W is a (C₁-C₄)alkyl,
   is treated with a cyclising agent, and
2) where appropriate, a radical R in a compound of formula (I) is transformed into another radical R, thereby forming another compound of formula (I), and/or a compound of formula (I) is converted into one of its pharmaceutically acceptable salts by being treated with an acid or base.

Unless expressly indicated otherwise, the radicals and/or parameters B, R, R¹ to R⁶, X, -Y-Z-, and halogen have, both above and below, the meanings indicated in association with the above defined formula (I).

The radical X is preferably -CH₂-, -CO-, -O-, -NH-, and, furthermore, -NR"'- or -S-.

B is preferably CH, but also N.

The group -Y-Z- is preferably 4-R¹-5-R²-thiophen-2,3-diyl, and, furthermore, preferably 2-R¹-5-R²-thiophen-3,4-diyl or 3-R¹-2-R²-thiophen-4,5-diyl.

In one preferred embodiment of the present invention R is preferably H, and, furthermore, preferably unsubstituted or monosubstituted phenoxy, specifically and preferably o-, m- or p-methylphenoxy, o-, m- or p-methoxyphenoxy, o-, m- or p-fluorophenoxy, o-, m- or p-chlorophenoxy, and, in addition, preferably o-, m- or p-trifluoromethylphenoxy.

In an other preferred embodiment of the invention, R is preferably unsubstituted or monosubstituted benzyl, specifically and preferably o-, m- or p-methylbenzyl, o-, m- or p-methoxybenzyl, o-, m- or p-fluorobenzyl, o-, m- or p-chlorobenzyl, and, in addition, preferably o-, m- or p-trifluoromethylbenzyl. Furthermore, R is preferably unsubstituted or monosubstituted benzoyl, specifically and preferably o-, m- or p-methylbenzoyl, o-, m- or p-methoxybenzoyl, o-, m- or p-fluorobenzoyl, o-, m- or p-chlorobenzoyl, and, in addition, preferably o-, m- or p-triflouromethylbenzoyl. Furthermore, R is preferably unsubstituted or monosubstituted anilino, specifically and preferably o-, m- or p-methylanilino, o-, m- or p-methoxyanilino, o-, m- or p-fluoroanilino, o-, m- or p-chloroanilino, and, in addition, preferably o-, m- or p-trifluoromethylanilino.

In addition, R is preferably o-, m- or p-nitrophenoxy, o-, m- or p-N,N-dimethylaminophenoxy, o-, m- or p-acetamidophenoxy, o-, m- or p-nitrobenzyl, o-, m- or p-N,N-dimethylaminobenzyl, o-, m- or p-acetamidobenzyl, o-, m- or p-nitrobenzoyl, o-, m- or p-N,N-dimethylaminobenzoyl, o-, m- or p-acetamidobenzoyl, o-, m- or p-nitroanilino, o-, m- or p-N,N-dimethylaminoanilino or o-, m- or p-acetamidoanilino.

R¹, R² and R⁵ are preferably H, methyl, ethyl, chloro, and, in addition, propyl or bromo, while R³ and R⁴ are in each case preferably H, and, in addition, methyl, methoxy, fluoro, chloro or trifluoromethyl.

R⁶ is preferably H or F, and, in addition Br.

Some preferred groups of compounds are expressed by the following partial formulae (Ia) to (Ie), which correspond to formula (I) in which the radicals which are not designated in more detail have the meanings indicated in association with formula (I), but in which:
in (Ia), B is CH;
in (Ib), B is N;
in (Ic), -Y-Z- is 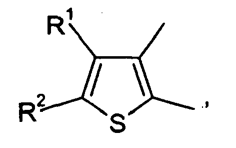 wherein
   R¹ is H or CH₃,
   R² is H, CH₃, C₂H₅, Cl or Br,
   R³ is H, CH₃, OH, OCH₃, F, Cl, CF₃, NO₂, NH₂, N(CH₃)₂ or NHCOCH₃,
   R⁴ is H,
   R⁵ is H and
   X is -CH₂-, -CO-, -O- or -NH-;
in (Id), -Y-Z- is 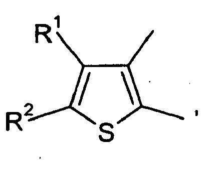 R is H or 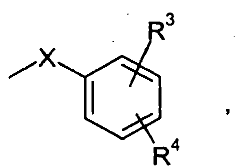 R¹ is H or CH₃,
   R² is H, CH₃, C₂H₅, Cl or Br,
   R³ is H, CH₃, OCH₃, F, Cl or CF₃,
   R⁴ is H, and
   X is -CH₂-, -CO-, -O- or -NH-;
in (le), B is CH,
   -Y-Z- is 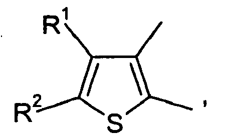 RisH,
   R¹ is H or CH₃, and
   R² is H, CH₃, C₂H₅, Cl or Br.

An other preferred embodiment of the present invention relates to compounds of formula (I) (formula (If)) where:
B is CH,
R and R⁶ are each H,
-Y-Z- is 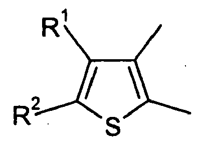
wherein
R¹ is H, methyl, ethyl, isopropyl, isobutyl, cyclopropyl,
R² is H, Cl, Br or methyl,
or,
R¹ and R² together form the group -(CH₂)₄-.

Preferred compounds of formula (If) are:
- 4-Hydroxy-2,3-dimethyl-5-phenyl-7*H*-thieno[2,3*-b*]pyridin-6-one;
- 2-Ethyl-4-hydroxy-5-phenyl-7*H-*thieno[2,3*-b*]pyridin-6-one;
- 2-Bromo-4-hydroxy-3-methyl-5-phenyl-7*H-*thieno[2,3*-b*]pyridin-6-one;
- 2-Bromo-4-hydroxy-5-phenyl-7*H-*thieno[2,3*-b*]pyridin-6-one;
- 2-Chloro-4-hydroxy-3-isopropyl-5-phenyl-7H-thieno[2,3-b]pyridin-6-one;
- 3-sec-Butyl-2-chloro-4-hydroxy-5-phenyl-7*H-*thieno[2,3*-b*]pyridin-6-one;
- 4-Hydroxy-3-isopropyl-5-phenyl-7*H*-thieno[2,3*-b*]pyridin-6-one;
- 3-sec-Butyl-4-hydroxy-5-phenyl-7H-thieno[2,3-b]pyridin-6-one; and
- 2-chloro-3-cyclopropyl-4-hydroxy-5-(3-phenoxy-phenyl)-7H-thieno[2,3-b]pyridine-6-one.

An other preferred embodiment of the present invention relates to new compounds of formula (I) (formula (Ig)) wherein:
B is CH,
R and R⁶ are each H,
-Y-Z- is 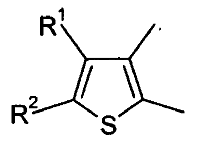
in which
R¹ is cyclopropyl,
R² is H, or
R¹ and R² together form-(CH₂)₄-.

Preferred compounds of formula (Ig) are:
- 4-Hydroxy-3-phenyl-5,6,7,8-tetrahydro-1*H*-benzo[4,5]thieno[2,3-*b*]pyridin-2-one, and
- 3-Cyclopropyl-4-hydroxy-5-phenyl-7*H*-thieno[2,3*-b*]pyridin-6-one.

Compounds of formula(I), as well as the starting compounds for their preparation, are otherwise prepared by methods which are known *per se,* as described in the literature (for example in the standard works such as Houben-Weyl, "Methoden der organischen Chemie" [Methods of organic chemistry], Georg-Thieme-Verlag, Stuttgart; in particular, however, in Journal of Medicinal Chemistry, (1994), 37, 1402-1405), and specifically under reaction conditions which are known, and are suitable, for the said reactions. In this context, use can also be made of variants which are known per se but which are not mentioned here in more detail.

If desired, the starting compounds can also be formed *in situ,* so that they are not isolated from the reaction mixture but are instead immediately subjected to further reaction to form the compounds of the formula (I).

The compounds of formula (I) are, for example, obtained by treating a compound of formula (II) with a cyclising agent, preferably a base. Examples of bases which may be used are potassium or sodium alcoholate, such as potassium or sodium methoxide, ethoxide or tert-butoxide, in an inert solvent, preferably the underlying alcohol, NaH in dimethylformamide (DMF) or KN[Si(CH₃)₃]₂ in an inert solvent.

The cyclisation reaction is expediently carried out at temperatures of between about -100°C and about +160°C; it is preferably carried out at a temperature of between -85°C and +50°C.

Suitable inert solvents are, in particular, alcohols, such as methanol, ethanol, isopropanol, n-butanol or *tert*-butanol,; ethers, such as diethyl ether, diisopropyl ether, tetrahydrofuran (THF) or dioxane; glycol ethers, such as ethylene glycol monomethyl ether or ethylene glycol monoethyl ether (methyl glycol or ethyl glycol) or ethylene glycol dimethyl ether (diglyme); ketones, such as acetone or butanone; nitriles, such as acetonitrile; nitro compounds, such as nitromethane or nitrobenzene; carboxylic acids, such as formic acid or acetic acid; esters, such as ethyl acetate; amides, such as DMF, dimethylacetamide or hexamethyl phosphoric triamide (HMPT); sulphoxides, such as dimethyl sulphoxide; carbon disulphide; chlorinated hydrocarbons, such as methylene chloride, chloroform, trichloroethylene, 1,2-dichloroethane or carbon tetrachloride; and hydrocarbons, such as hexane, petroleum ether, benzene, toluene or xylene. Mixtures of these solvents with each other are also suitable.

In the compounds of the formula (II), W is preferably methyl, and, in addition, ethyl.

The starting compounds of formula (II) may be prepared by methods which are known *per* se. Thus, for example, methyl 2-amino-4-methylthiophene-3-carboxylate is reacted with, for example, phenylacetyl chloride in an inert solvent, resulting in the formation of methyl 2-phenylacetamido-4-methylthiophene-3-carboxylate. This is expediently carried out at temperatures of between about 0°C and about 200°C; the reaction is preferably carried out at between 60°C and 90°C.

It is furthermore possible to convert a compound of formula (I) into another compound of the formula (I) by converting the radical R into another radical, for example by reducing nitro groups (for example by hydrogenating on Raney nickel or Pd/charcoal in an inert solvent such as methanol or ethanol) to form amino groups, and/or functionally modifying free amino and/or hydroxyl groups, and/or liberating functionally modified amino and/or hydroxyl groups by solvolysis or hydrogenolysis.

Thus, free amino groups can, for example, be acylated, in a customary manner, with an acid chloride or acid anhydride, or alkylated with an unsubstituted or substituted alkyl halide, expediently in an inert solvent such as dichloromethane or THF, and/or in the presence of a base such as triethylamine or pyridine, at temperatures of between -60°C and +30°C. Free hydroxyl groups can be alkylated in an analogous manner.

If desired, a functionally modified amino and/or hydroxyl group in a compound of the formula (I) can be liberated by solvolysis or hydrogenolysis using customary methods. Thus, a compound of the formula (I), in which R represents -NHCOA or -OA, can, for example, be converted into the corresponding compound of formula (I) in which R represents -NH₂ or -OH.

Here-above, A represents a linear or branched lower alkyl, for example methyl or ethyl.

Acylated amines of the formula (I) in which the phenyl radical or pyridyl radical is substituted once by -NHCOA can be cleaved, resulting in the formation of the corresponding amino derivatives. For example, the acylamino compounds can be cleaved by treating them with methanolic potassium hydroxide solution at a temperature comprised between about 20°C to 140°C.

Ethers of the formula (I) in which the phenyl radical or pyridyl radical is substituted once by -O-A can be cleaved, resulting in the formation of the corresponding hydroxyl derivatives. For example, the ethers can be cleaved by treating them with dimethyl sulphide/boron tribromide complex, for example in toluene, ethers such as THF, or dimethyl sulphoxide, or by fusing them with pyridine hydrohalides or aniline hydrohalides, preferably pyridine hydrochloride, at about 150°C-250°C, or by treating them with diisobutylaluminium hydride in toluene at about 0°C-110°C.

A base of the formula (I) can be converted with an acid into the affiliated acid addition salt, for example by reacting equivalent quantities of the base and of the acid in an inert solvent such as ethanol, and then evaporating. Acids which yield physiologically harmless salts are particularly suitable for this reaction. Thus, inorganic acids which can be used are, for example, chosen from among sulphuric acid, nitric acid, hydrohalic acids, such as hydrochloric acid or hydrobromic acid, phosphoric acids, such as orthophosphoric acid, and sulphamic acid, and, in addition, organic acids, in particular aliphatic, alicyclic, araliphatic, aromatic or heterocyclic monobasic or polybasic carboxylic, sulphonic or sulphuric acids, for example formic acid, acetic acid, propionic acid, pivalic acid, diethylacetic acid, malonic acid, succinic acid, pimelic acid, fumaric acid, maleic acid, lactic acid, tartaric acid, malic acid, citric acid, gluconic acid, ascorbic acid, nicotinic acid, isonicotinic acid, methanesulphonic acid, ethanesulphonic acid, ethanedisulphonic acid, 2-hydroxyethanesulphonic acid, benzenesulphonic acid, p-toluenesulphonic acid, naphthalenemonosulphonic acid, naphthalenedisulphonic acid and laurylsulphuric acid. Salts with physiologically harmful acids, for example picrates, can be used to isolate and/or purify the compounds of formula (I).

On the other hand, compounds of the formula (I) can be converted with bases (for example sodium hydroxide or potassium hydroxide or sodium carbonate or potassium carbonate) into the corresponding metal salts, in particular alkali metal salts or alkaline earth metal salts, or into the corresponding ammonium salts.

The invention also relates to the use of the compounds of the formula (I), and their physiologically harmless salts, for producing pharmaceutical preparations, in particular by a non-chemical route. In this context, they can be brought into a suitable dosage form together with at least one solid, liquid and/or semi-liquid carrier and/or auxiliary substance and, where appropriate, in combination with one or more additional active compounds.

The invention furthermore relates to pharmaceutical preparations which contain at least one compound of the formula (I) and/or one of its physiologically harmless salts.

These preparations can be used as medicaments in human or veterinary medicine. Suitable carrier substances are organic or inorganic substances which are suitable for enteral (for example oral), parenteral or topical administration and which do not react with the novel compounds, for example water, vegetable oils, benzyl alcohols, alkylene glycols, polyethylene glycols, glycerol triacetate, gelatin, carbohydrates such as lactose or starch, magnesium stearate, talc or vaseline. Tablets, pills, coated tablets, capsules, powders, granules, syrups, juices or drops serve, in particular, for oral use, suppositories serve for rectal use, solutions, preferably oily or aqueous solutions, and, in addition, suspensions, emulsions or implants, serve for parenteral use, while ointments, creams or powders serve for topical use.

The compounds of the present invention can also be lyophilized, and the resulting lyophilisates can be used, for example for preparing injection preparations. The listed preparations can be sterilized and/or contain auxiliary substances such as lubricants, preservatives, stabilizers and/or wetting agents, emulsifiers, salts for influencing the osmotic pressure, buffer substances, dyes, flavourings and/or aromatizing agents. If desired, they can also contain one or more additional active compounds, for example, one or more vitamins.

The compounds of the formula (I), and their physiologically harmless salts, can be used in the control of diseases, in particular conditions related to glucose homeostasia and energy expenditure. The compounds are particularly suitable for treating diabetes, metabolic syndrome, related disorders and obesity.

In this context, the substances (compounds of the present invention) are, as a rule, preferably administered in doses of between about 1 mg and 500 mg, in particular of between 5 mg and 100 mg, per dosage unit. The daily dose is preferably between about 0.02 mg/kg and 10 mg/kg of body weight. However, the specific dose for each particular patient depends on a wide variety of factors, for example on the activity of the specific compound employed, on the age, body weight, general state of health and sex, on the nutrition, on the time and route of administration, on the speed of excretion, on the drug combination and on the severity of the particular disease to which the therapy applies. Oral administration is preferred.

Both above and below, all temperatures are given in °C. In the following examples, "customary working-up" denotes: if necessary, water is added, if necessary, depending on the constitution of the end product, the pH of the mixture is adjusted to pH values of between 2 and 10, and the mixture is then extracted with ethyl acetate or dichloromethane; the organic phase is then separated off, dried over sodium sulphate and evaporated, and the residue is purified by chromatography on silica gel and/or by crystallization.

Rf values correspond to Rf values obtained from thin layer chromatography on silica gel.

m.p. stands for "melting point".

The invention is further illustrated by the following representatives and non-limiting examples:

### Example 1

A solution of 0.6 g of methyl 5-chloro-4-methyl-2-(phenylaceta-mido)thiophene-3-carboxylate (m.p.: 105-107°; obtainable by chlorinating the corresponding chlorine-free compound with N-chlorosuccinimide) in 20 mL of THF is cooled down to -70°. 8.16 mL of a 0.5 molar solution of KN(Si(CH₃)₃)₂ in toluene are then added dropwise. The reaction solution is slowly brought to room temperature and then evaporated. The residue is taken up in water, and this solution is extracted several times with diethyl ether. The aqueous phase is then acidified with 2 N HCl, and the precipitate is filtered off, washed with water and, after having been digested with diethyl ether, filtered off once again and dried. 2-Chloro-3-methyl-4-hydroxy-5-phenyl-6,7-dihydro-thieno[2,3-b]pyridin-6-one is obtained.

Rf (CH₂Cl₂/MeOH 10:1): 0.43.

### Examples 2 to 8

The following 2-R²-3-R¹-4-hydroxy-5-phenyl-6,7-dihydrothieno[2,3-b] pyridin-6-ones:

| ***Ex.*** | ***R¹*** | ***R²*** | ***Rf** (CH₂Cl₂*/*MeOH 10:1)* |
|---|---|---|---|
| 2 | Me | H | 0.43 |
| 3 | H | Me | 0.36 |
| 4 | Me | Me | 0.49 |
| 5 | H | Et | 0.44 |
| 6 | H | H | 0.31 |
| 7 | Me | Br | 0.38 |
| 8 | H | Br | 0.34 |

are obtained, in analogy with Example 1, from the following methyl or ethyl 2-phenylacetamido-4-R¹-5-R²-thiophene-3-carboxylates, respectively:

| ***Precursor of Ex.*** | ***R¹*** | ***R²*** | ***A*** | ***M.p.*** |
|---|---|---|---|---|
| 2 | Me | H | Me | 105.5-106.5° |
| 3 | H | Me | Me | 110.5-111.5° |
| 4 | Me | Me | Et | 84-86° |
| 5 | H | Et | Me | 105-106° |
| 6 | H | H | Me | 90-92° |
| 7 | Me | Br | Me | 105-106.5° |
| 8 | H | Br | Me | - |

In the above table, A stands for the methyl or ethyl ester group.

### Examples 9 to 72

The following 2-chloro-3-methyl-4-hydroxy-5-(3-R-phenyl)-6,7-dihydro-thieno[2,3-b]pyridin-6-ones:

| ***Ex.*** | ***R*** |
|---|---|
| 9 | phenoxy, Rf 0.44 |
| 10 | o-methylphenxoy |
| 11 | m-methylphenoxy |
| 12 | p-methylphenoxy |
| 13 | o-methoxyphenoxy |
| 14 | m-methoxyphenoxy |
| 15 | p-methoxyphenoxy |
| 16 | o-fluorophenoxy |
| 17 | m-fluorophenoxy |
| 18 | p-fluorophenoxy |
| 19 | o-chlorophenoxy |
| 20 | m-chlorophenoxy |
| 21 | p-chlorophenoxy |
| 22 | o-trifluoromethylphenoxy |
| 23 | m-trifluoromethylphenoxy |
| 24 | p-trifluoromethylphenoxy |
| 25 | benzyl |
| 26 | o-methylbenzyl |
| 27 | m-methylbenzyl |
| 28 | p-methylbenzyl |
| 29 | o-methoxybenzyl |
| 30 | m-methoxybenzyl |
| 31 | p-methoxybenzyl |
| 32 | o-fluorobenzyl |
| 33 | m-fluorobenzyl |
| 34 | p-fluorobenzyl |
| 35 | o-chlorobenzyl |
| 36 | m-chlorobenzyl |
| 37 | p-chlorobenzyl |
| 38 | o-trifluoromethylbenzyl |
| 39 | m-trifluoromethylbenzyl |
| 40 | p-trifluoromethylbenzyl |
| 41 | benzoyl |
| 42 | o-methylbenzoyl |
| 43 | m-methylbenzoyl |
| 44 | p-methylbenzoyl |
| 45 | o-methoxybenzoyl |
| 46 | m-methoxybenzoyl |
| 47 | p-methoxybenzoyl |
| 48 | o-fluorobenzoyl |
| 49 | m-fluorobenzoyl |
| 50 | p-fluorobenzoyl |
| 51 | o-trifluoromethylbenzoyl |
| 52 | m-trifluoromethylbenzoyl |
| 53 | p-trifluoromethylbenzoyl |
| 54 | o-chlorobenzoyl |
| 55 | m-chlorobenzoyl |
| 56 | p-chlorobenzoyl |
| 57 | anilino |
| 58 | o-methylanilino |
| 59 | m-methylanilino |
| 60 | p-methylanilino |
| 61 | o-methoxyanilino |
| 62 | m-methoxyanilino |
| 63 | p-methoxyanilino |
| 64 | o-fluoroanilino |
| 65 | m-fluoroanilino |
| 66 | p-fluoroanilino |
| 67 | o-chloroanilino |
| 68 | m-chloroanilino |
| 69 | p-chloroanilino |
| 70 | o-trifluoromethylanilino |
| 71 | m-trifluoromethylanilino |
| 72 | p-trifluoromethylanilino |

are obtained, in analogy with Example 1, from the following methyl 2-(3-R-phenyl)acetamido-4-methyl-5-chlorothiophene-3-carboxylates:

| ***Precursor of Ex.*** | ***R*** |
|---|---|
| 9 | phenoxy, m.p. 113-115° |
| 10 | o-methylphenoxy |
| 11 | m-methylphenoxy |
| 12 | p-methylphenoxy |
| 13 | o-methoxyphenoxy |
| 14 | m-methoxyphenoxy |
| 15 | p-methoxyphenoxy |
| 16 | o-fluorophenoxy |
| 17 | m-fluorophenoxy |
| 18 | p-fluorophenoxy |
| 19 | o-chlorophenoxy |
| 20 | m-chlorophenoxy |
| 21 | p-chlorophenoxy |
| 22 | o-trifluoromethylphenoxy |
| 23 | m-trifluoromethylphenoxy |
| 24 | p-trifluoromethylphenoxy |
| 25 | benzyl |
| 26 | o-methylbenzyl |
| 27 | m-methylbenzyl |
| 28 | p-methylbenzyl |
| 29 | o-methoxybenzyl |
| 30 | m-methoxybenzyl |
| 31 | p-methoxybenzyl |
| 32 | o-fluorobenzyl |
| 33 | m-fluorobenzyl |
| 34 | p-fluorobenzyl |
| 35 | o-chlorobenzyl |
| 36 | m-chlorobenzyl |
| 37 | p-chlorobenzyl |
| 38 | o-trifluoromethylbenzyl |
| 39 | m-trifluoromethylbenzyl |
| 40 | p-trifluoromethylbenzyl |
| 41 | benzoyl |
| 42 | o-methylbenzoyl |
| 43 | m-methylbenzoyl |
| 44 | p-methylbenzoyl |
| 45 | o-methoxybenzoyl |
| 46 | m-methoxybenzoyl |
| 47 | p-methoxybenzoyl |
| 48 | o-fluorobenzoyl |
| 49 | m-fluorobenzoyl |
| 50 | p-fluorobenzoyl |
| 51 | o-trifluoromethylbenzoyl |
| 52 | m-trifluoromethylbenzyl |
| 53 | p-trifluoromethylbenzoyl |
| 54 | o-chlorobenzoyl |
| 55 | m-chlorobenzoyl |
| 56 | p-chlorobenzoyl |
| 57 | anilino |
| 58 | o-methylanilino |
| 59 | m-methylanilino |
| 60 | p-methylanilino |
| 61 | o-methoxyanilino |
| 62 | m-methoxyanilino |
| 63 | p-methoxyanilino |
| 64 | o-fluoroanilino |
| 65 | m-fluoroanilino |
| 66 | p-fluoroanilino |
| 67 | o-chloroanilino |
| 68 | m-chloroanilino |
| 69 | p-chloroanilino |
| 70 | o-trifluoromethylanilino |
| 71 | m-trifluoromethylanilino |
| 72 | p-trifluoromethylanilino. |

### Examples 73 to 136

The following 2-chloro-3-methyl-4-hydroxy-5-(4-R-2-pyridyl)-6,7-dihydrothieno[2,3-b]pyridin-6-ones:

| ***Ex.*** | ***R*** |
|---|---|
| 73 | phenoxy |
| 74 | o-methylphenoxy |
| 75 | m-methylphenoxy |
| 76 | p-methylphenoxy |
| 77 | o-methoxyphenoxy |
| 78 | m-methoxyphenoxy |
| 79 | p-methoxyphenoxy |
| 80 | o-fluorophenoxy |
| 81 | m-fluorophenoxy |
| 82 | p-fluorophenoxy |
| 83 | o-chlorophenoxy |
| 84 | m-chlorophenoxy |
| 85 | p-chlorophenoxy |
| 86 | o-trifluoromethylphenoxy |
| 87 | m-trifluoromethylphenoxy |
| 88 | p-trifluoromethylphenoxy |
| 89 | benzyl |
| 90 | o-methylbenzyl |
| 91 | m-methylbenzyl |
| 92 | p-methylbenzyl |
| 93 | o-methoxybenzyl |
| 94 | m-methoxybenzyl |
| 95 | p-methoxybenzyl |
| 96 | o-fluorobenzyl |
| 97 | m-fluorobenzyl |
| 98 | p-fluorobenzyl |
| 99 | o-chlorobenzyl |
| 100 | m-chlorobenzyl |
| 101 | p-chlorobenzyl |
| 102 | o-trifluoromethylbenzyl |
| 103 | m-trifluoromethylbenzyl |
| 104 | p-trifluoromethylbenzyl |
| 105 | benzoyl |
| 106 | o-methylbenzoyl |
| 107 | m-methylbenzoyl |
| 108 | p-methylbenzoyl |
| 109 | o-methoxybenzoyl |
| 110 | m-methoxybenzoyl |
| 111 | p-methoxybenzoyl |
| 112 | o-fluorobenzoyl |
| 113 | m-fluorobenzoyl |
| 114 | p-fluorobenzoyl |
| 115 | o-trifluoromethylbenzoyl |
| 116 | m-trifluoromethylbenzoyl |
| 117 | p-trifluoromethylbenzoyl |
| 118 | o-chlorobenzoyl |
| 119 | m-chlorobenzoyl |
| 120 | p-chlorobenzoyl |
| 121 | anilino |
| 122 | o-methylanilino |
| 123 | m-methylanilino |
| 124 | p-methylanilino |
| 125 | o-methoxyanilino |
| 126 | m-methoxyanilino |
| 127 | p-methoxyanilino |
| 128 | o-fluoroanilino |
| 129 | m-fluoroanilino |
| 130 | p-fluoroanilino |
| 131 | o-chloroanilino |
| 132 | m-chloroanilino |
| 133 | p-chloroanilino |
| 134 | o-trifluoromethylanilino |
| 135 | m-trifluoromethylanilino |
| 136 | p-trifluoromethylanilino |

are obtained, in analogy with Example 1, from the following methyl 2-(4-R-2-pyridyl)acetamido-4-methyl-5-chlorothiophene-3-carboxylates:

| ***Precursor of Ex.*** | ***R*** |
|---|---|
| 73 | phenoxy |
| 74 | o-methylphenoxy |
| 75 | m-methylphenoxy |
| 76 | p-methylphenoxy |
| 77 | o-methoxyphenoxy |
| 78 | m-methoxyphenoxy |
| 79 | p-methoxyphenoxy |
| 80 | o-fluorophenoxy |
| 81 | m-fluorophenoxy |
| 82 | p-fluorophenoxy |
| 83 | o-chlorophenoxy |
| 84 | m-chlorophenoxy |
| 85 | p-chlorophenoxy |
| 86 | o-trifluoromethylphenoxy |
| 87 | m-trifluoromethylphenoxy |
| 88 | p-trifluoromethylphenoxy |
| 89 | benzyl |
| 90 | o-methylbenzyl |
| 91 | m-methylbenzyl |
| 92 | p-methylbenzyl |
| 93 | o-methoxybenzyl |
| 94 | m-methoxybenzyl |
| 95 | p-methoxybenzyl |
| 96 | o-fluorobenzyl |
| 97 | m-fluorobenzyl |
| 98 | p-fluorobenzyl |
| 99 | o-chlorobenzyl |
| 100 | m-chlorobenzyl |
| 101 | p-chlorobenzyl |
| 102 | o-trifluoromethylbenzyl |
| 103 | m-trifluoromethylbenzyl |
| 104 | p-trifluoromethylbenzyl |
| 105 | benzoyl |
| 106 | o-methylbenzoyl |
| 107 | m-methylbenzoyl |
| 108 | p-methylbenzoyl |
| 109 | o-methoxybenzoyl |
| 110 | m-methoxybenzoyl |
| 111 | p-methoxybenzoyl |
| 112 | o-fluorobenzoyl |
| 113 | m-fluorobenzoyl |
| 114 | p-fluorobenzoyl |
| 115 | o-trifluoromethylbenzoyl |
| 116 | m-trifluoromethylbenzoyl |
| 117 | p-trifluoromethylbenzoyl |
| 118 | o-chlorobenzoyl |
| 119 | m-chlorobenzoyl |
| 120 | p-chlorobenzoyl |
| 121 | anilino |
| 122 | o-methylanilino |
| 123 | m-methylanilino |
| 124 | p-methylanilino |
| 125 | o-methoxyanilino |
| 126 | m-methoxyanilino |
| 127 | p-methoxyanilino |
| 128 | o-fluoroanilino |
| 129 | m-fluoroanilino |
| 130 | p-fluoroanilino |
| 131 | o-chloroanilino |
| 132 | m-chloroanilino |
| 133 | p-chloroanilino |
| 134 | o-trifluoromethylanilino |
| 135 | m-trifluoromethylanilino |
| 136 | p-trifluoromethylanilino |

### Examples 137 to 143

The following 2-R²-3-R¹-4-hydroxy-5-(2-pyridyl)-6,7-dihydrothieno[2,3-b]pyridin-6-ones:

| ***Ex.*** | ***R¹*** | ***R²*** |
|---|---|---|
| 137 | Me | H |
| 138 | H | Me |
| 139 | Me | Me |
| 140 | H | Et |
| 141 | H | H |
| 142 | Me | Br |
| 143 | H | Br |

are obtained, in analogy with Example 1, from the following methyl 2-(2-pyridyl)acetamido-4-R¹-5-R²-thiophene-3-carboxylates:

| ***Precursor of Ex.*** | ***R¹*** | ***R²*** |
|---|---|---|
| 137 | Me | H |
| 138 | H | Me |
| 139 | Me | Me |
| 140 | H | Et |
| 141 | H | H |
| 142 | Me | Br |
| 143 | H | Br |

### Examples 144-147

The following 2-chloro-3-methyl-4-hydroxy-5-(3-R-phenyl)-6,7-dihydrothieno[2,3-b]pyridin-6-ones:

| ***Ex.*** | ***R*** |
|---|---|
| 144 | 2-thienylcarbonyl |
| 145 | 2-thienylmethyl |
| 146 | 3-thienylcarbonyl |
| 147 | 3-thienylmethyl |

are obtained, in analogy with Example 1, from the following methyl 2-(3-R-phenyl)acetamido-4-methyl-5-chlorothiophene-3-carboxylates:

| ***Precursor of Ex*.** | ***R*** |
|---|---|
| 144 | 2-thienylcarbonyl |
| 145 | 2-thienylmethyl |
| 146 | 3-thienylcarbonyl |
| 147 | 3-thienylmethyl |

### Examples 148-183

The following 2-chloro-3-methyl-4-hydroxy-5-(3-R-phenyl)-6,7-dihydro-thieno[2,3-b]pyridin-6-ones:

| ***Ex.*** | ***R*** |
|---|---|
| 148 | o-nitrophenoxy |
| 149 | m-nitrophenoxy |
| 150 | p-nitrophenoxy |
| 151 | o-N,N-dimethylaminophenoxy |
| 152 | m-N,N-dimethylaminophenoxy |
| 153 | p-N,N-dimethylaminophenoxy |
| 154 | o-acetamidophenoxy |
| 155 | m-acetamidophenoxy |
| 156 | p-acetamidophenoxy |
| 157 | o-nitrobenzyl |
| 158 | m-nitrobenzyl |
| 159 | p-nitrobenzyl |
| 160 | o-N,N-dimethylaminobenzyl |
| 161 | m-N,N-dimethylaminobenzyl |
| 162 | p-N,N-dimethylaminobenzyl |
| 163 | o-acetamidobenzyl |
| 164 | m-acetamidobenzyl |
| 165 | p-acetamidobenzyl |
| 166 | o-nitrobenzoyl |
| 167 | m-nitrobenzoyl |
| 168 | p-nitrobenzoyl |
| 169 | o-N,N-dimethylaminobenzoyl |
| 170 | m-N,N-dimethylaminobenzoyl |
| 171 | p-N,N-dimethylaminobenzoyl |
| 172 | o-acetamidobenzoyl |
| 173 | m-acetamidobenzoyl |
| 174 | p-acetamidobenzoyl |
| 175 | o-nitroanilino |
| 176 | m-nitroanilino |
| 177 | p-nitroanilino |
| 178 | o-N,N-dimethylaminoanilino |
| 179 | m-N,N-dimethylaminoanilino |
| 180 | p-N,N-dimethylaminoanilino |
| 181 | o-acetamidoanilino |
| 182 | m-acetamidoanilino |
| 183 | p-acetamidoanilino |

are obtained, in analogy with Example 1, from the following methyl 2-(3-R-phenyl)acetamido-4-methyl-5-chlorothiophene-3-carboxylates:

| ***Precursor of Ex.*** | ***R*** |
|---|---|
| 148 | o-nitrophenoxy |
| 149 | m-nitrophenoxy |
| 150 | p-nitrophenoxy |
| 151 | o-N,N-dimethylaminophenoxy |
| 152 | m-N,N-dimethylaminophenoxy |
| 153 | p-N,N-dimethylaminophenoxy |
| 154 | o-acetamidophenoxy |
| 155 | m-acetamidophenoxy |
| 156 | p-acetamidophenoxy |
| 157 | o-nitrobenzyl |
| 158 | m-nitrobenzyl |
| 159 | p-nitrobenzyl |
| 160 | o-N,N-dimethylaminobenzyl |
| 161 | m-N,N-dimethylaminobenzyl |
| 162 | p-N,N-dimethylaminobenzyl |
| 163 | o-acetamidobenzyl |
| 164 | m-acetamidobenzyl |
| 165 | p-acetamidobenzyl |
| 166 | o-nitrobenzoyl |
| 167 | m-nitrobenzoyl |
| 168 | p-nitrobenzoyl |
| 169 | o-N,N-dimethylaminobenzoyl |
| 170 | m-N,N-dimethylaminobenzoyl |
| 171 | p-N,N-dimethylaminobenzoyl |
| 172 | o-acetamidobenzoyl |
| 173 | m-acetamidobenzoyl |
| 174 | p-acetamidobenzoyl |
| 175 | o-nitroanilino |
| 176 | m-nitroanilino |
| 177 | p-nitroanilino |
| 178 | o-N,N-dimethylaminoanilino |
| 179 | m-N,N-dimethylaminoanilino |
| 180 | p-N,N-dimethylaminoanilino |
| 181 | o-acetamidoanilino |
| 182 | m-acetamidoanilino |
| 183 | p-acetamidoanilino. |

### Example 184

7-Hydroxy-6-phenyl-4,5-dihydrothieno-[3,2-b]-pyridin-5-one (R_{f} 0.31, dichloromethane/methanol 10:1), is obtained, in analogy with Example 1, by cyclisation, from methyl 3-phenylacetamidothiophene-2-carboxylate.

### Example 185

The compound 4-hydroxy-3-phenyl-1,2-dihydrothieno[3,4-b]pyridin-2-one (R_{f} 0.37, dichloromethane/ methanol 10:1), is obtained, in analogy with Example 1, from methyl 4-phenylacetamidothiophene-3-carboxylate.

### Example 186

20 mg of platinum oxide are added to a solution of 4.28 g of 2-chloro-3-methyl-4-hydroxy-5-(3-m-nitrophenoxyphenyl)-6,7-dihydrothieno[2,3-b]pyridin-6-one in 50 mL of 95% ethanol. Hydrogen is passed through the solution until three molar equivalents have been absorbed. The platinum is filtered off and the alcohol is distilled off. 2-Chloro-3-methyl-4-hydroxy-5-(3-m-aminophenoxyphenyl)-6,7-dihydrothieno[2,3-b]pyridin-6-one is obtained.

### Example 187

A solution of 4.4 g of 2-chloro-3-methyl-4-hydroxy-5-(3-o-acetamidophenoxyphenyl)-6,7-dihydrothieno [2,3-b]pyridin-6-one in 80 mL of 10% methanolic KOH solution is boiled for 48 hours. 2-Chloro-3-methyl-4-hydroxy-5-(3-o-aminophenoxyphenyl)-6,7-dihydro-thieno[2,3-b]pyridin-6-one is obtained after the customary working-up.

### Example 188

A mixture of 4.25 g of 2-chloro-3-methyl-4-hydroxy-5-(3-p-methoxybenzoylphenyl)-6,7-dihydrothieno[2,3-b]pyridin-6-one and 3.5 g of pyridine hydrochloride is stirred at 160° for 3 hours. 2-Chloro-3-methyl-4-hydroxy-5-(3-p-hydroxybenzoylphenyl)-6,7-dihy-drothieno[2,3-b]pyridin-6-one is obtained after the customary working-up.

### Examples 189-199

The following 2-R²-3-R¹-4-hydroxy-5-(3-R-4-R⁶-phenyl)-6,7-dihydro-thieno[2,3-b]pyridin-6-ones:

| ***Ex.*** | ***R*** | ***R⁶*** | ***R¹*** | ***R²*** | ***Rf (CH₂Cl₂*/*MeOH 10:1)*** |
|---|---|---|---|---|---|
| 189 | H | F | H | H | 0.34 |
| 190 | H | H | Ethyl | H | 0.38 |
| 191 | H | H | Ethyl | Br | 0.35 |
| 192 | H | H | Ethyl | Cl | 0.4 |
| 193 | OC₆H₅ | H | Methyl | H | 0.39 |
| 194 | OC₆H₅ | H | Ethyl | Cl | 0.59 |
| 195 | H | H | CH(CH₃)₂ | Cl | 0.56 |
| 196 | OC₆H₅ | H | CH(CH₃)₂ | Cl | 0.58 |
| 197 | OC₆H₅ | H | cyclohexyl | Cl | 0.44 |
| 198 | OC₆H₅ | H | cyclopropyl | Cl | 0.42 |
| 199 | OC₆H₅ | H | tert.-butyl | Cl | 0.45 |

are obtained, in analogy with Example 1, from the following methyl-2-(3-R-4-R⁶-phenyl)acetamido-4-R¹-5-R²-thiophene-3-carboxylates:

| ***Precursor of Ex.*** | ***R*** | ***R⁶*** | ***R¹*** | ***R²*** | ***m.p.*** |
|---|---|---|---|---|---|
| 189 | H | F | H | H | 102.5-104° |
| 190 | H | H | Ethyl | H | 116-117° |
| 191 | H | H | Ethyl | Br | 92-93° |
| 192 | H | H | Ethyl | Cl | 89.5-91.5 |
| 193 | OC6H₅ | H | Methyl | H | 83-85° |
| 194 | OC₆H₅ | H | Ethyl | Cl | 116-118° |
| 195 | H | H | CH(CH₃)₂ | Cl | 70-71° |
| 196 | OC₆H₅ | H | CH(CH₃)₂ | Cl | 61-63° |
| 197 | OC₆H₅ | H | cyclohexyl | Cl | 83.5-84.5° |
| 198 | OC₆H₅ | H | cyclopropyl | Cl | 95-97° |
| 199 | OC₆H₅ | H | tert.-butyl | Cl | 63.5-66° |

### Examples 200-209

The following 2-R²-3-R¹-4-hydroxy-5-(3-R-4-R⁶-phenyl)-6,7-dihydro-thieno[2,3-b]pyridin-6-ones:

| ***Ex.*** | ***R*** | ***R⁶*** | ***R¹*** | ***R²*** |
|---|---|---|---|---|
| 200 | H | H | Methyl | Methyl |
| 201 | H | H | H | Ethyl |
| 202 | H | H | Mehyl | Br |
| 203 | H | H | H | Br |
| 204 | H | H | Isopropyl | Cl |
| 205 | OC₆H₅ | H | cyclopropyl | Cl |
| 206 | H | H | isobutyl | Cl |
| 207 | H | H | isopropyl | H |
| 208 | H | H | cyclopropyl | H |
| 209 | H | H | isobutyl | H |

are obtained, in analogy with Example 1, from the following methyl 2-(3-R-4-R⁶-phenyl)acetamido-4-R¹-5-R²-thiophene-3-carboxylates:

| ***Precursor of Ex.*** | ***R*** | ***R⁶*** | ***R¹*** | ***R²*** |
|---|---|---|---|---|
| 200 | H | H | Methyl | Methyl |
| 201 | H | H | H | Ethyl |
| 202 | H | H | Mehyl | Br |
| 203 | H | H | H | Br |
| 204 | H | H | Isopropyl | Cl |
| 205 | OC₆H₅ | H | cyclopropyl | Cl |
| 206 | H | H | isobutyl | Cl |
| 207 | H | H | isopropyl | H |
| 208 | H | H | cyclopropyl | H |
| 209 | H | H | isobutyl | H |

### Example 210

4-Hydroxy-3-phenyl-5,6,7,8-tetrahydro-1-benzothieno[2,3-b]pyridin-2(1*H*)-one is obtained, in analogy with example 1, by cyclisation, from ethyl 2-(phenylacetyl)amino-4,5,6,7-tetrahydro-1-benzothiophene-3-carboxylate

### Biological examples:

### Screening assay:

Human recombinant AMPK enzyme was expressed in *E*. Coli and was reactivated *in vitro* by LKB1 prior to enzyme activity determination.

AMPK enzyme activities were assayed by using 2 fluorescent based technologies (AlphaScreen / Delfia) and were carried out in microtiter plates (containing 25 mM Tris/HCl buffer, pH 7,5 with 100 µM ATP / 50 mM Hepes buffer, pH 7,4 with 125 µM ATP respectively) in the presence of a synthetic peptide substrate (AMARAASAAALARRR, the "AMARAA" peptide) and activators in serial dilutions. Reactions were initiated by the addition of AMPK (50-100 ng). Following mixing, the plates were incubated for 30 min at room temperature. Enzyme activity was assayed by using an anti-phosphoserine antibody to measure the quantity of phosphate incorporated into the AMARAA. Activity is expressed in % of control (basal activity):

| ***Compound number*** | ***structure*** | **α*-Screen*** | ***Delfia*** |
|---|---|---|---|
| 200 | 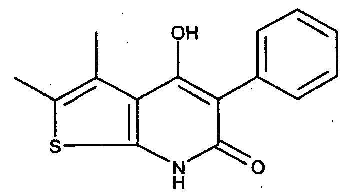 | 154 | 232 |
| 201 | 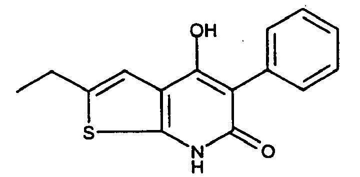 | 150 | 219 |
| 202 | 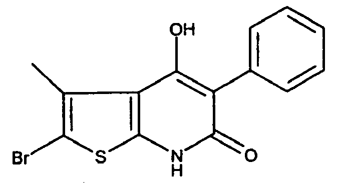 | 322 | 313 |
| 203 | 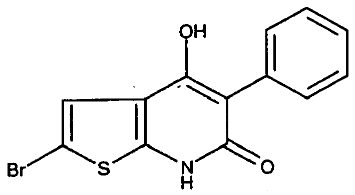 | 286 | 271 |
| 204 | 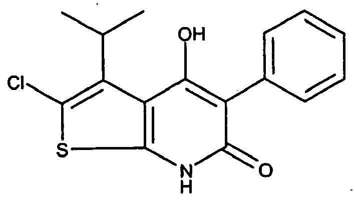 | 181 | 233 |
| 205 | 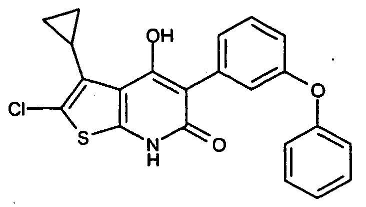 | 166 | 151 |
| 206 | 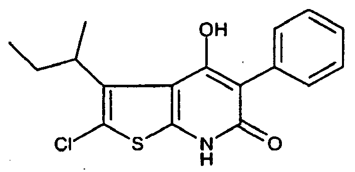 | 269 | 164 |
| 207 | 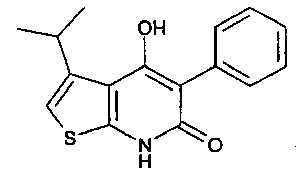 | 131 | 169 |
| 208 | 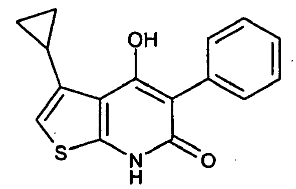 | 154 | 163 |
| 209 | 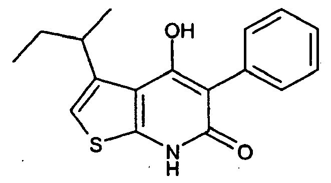 | 156 | 201 |
| 210 | 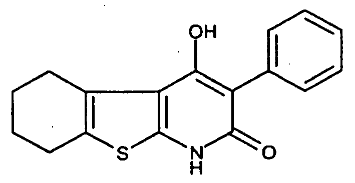 | 176 | 286 |

The following examples relate to pharmaceutical preparations which comprise active compounds of the formula I or their salts.

### Example A: Tablets and coated tablets

Tablets of the following composition, which, as required, are coated with a customary sugar coating surface on a sucrose base, are pressed in the customary manner:

| | |
|---|---|
| active compound of formula (I) | 100 mg |
| microcrystalline cellulose | 278.8 mg |
| lactose | 110 mg |
| corn starch | 11 mg |
| magnesium stearate | 5 mg |
| finely divided silicon dioxide | 0.2 mg |

### Example B: Hard gelatin capsules

Customary two-part hard gelatin capsules are in each case filled with

| | |
|---|---|
| active compound of formula (I) | 100 mg |
| lactose | 150 mg |
| cellulose | 50 mg |
| magnesium stearate | 6 mg |

### Example C: Soft gelatin capsules

Customary soft gelatin capsules are filled with a mixture comprising in each case 50 mg of active compound and 250 mg of olive oil.

### Example D: Ampoules

A solution of 200 g of active compound in 2 kg of 1,2-propanediol is made up to 10 L with water and used to fill ampoules such that each ampoule contains 20 mg of active compound.

### Example E: Aqueous suspension for oral administration

An aqueous suspension of the active compound is prepared in the customary manner. The single dose (5 mL) contains 100 mg of active compound, 100 mg of Na carboxymethylcellulose, 5 mg of Na benzoate and 100 mg of sorbitol.

## Claims

1. Use of a compound of formula (I): 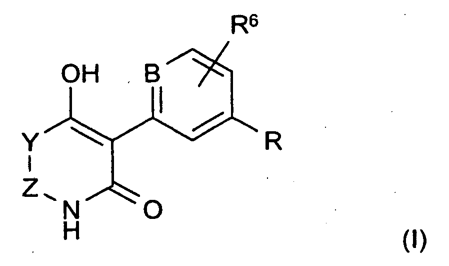 in which
B is CH or N, 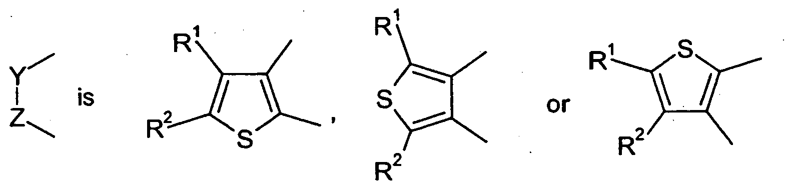 R is H, 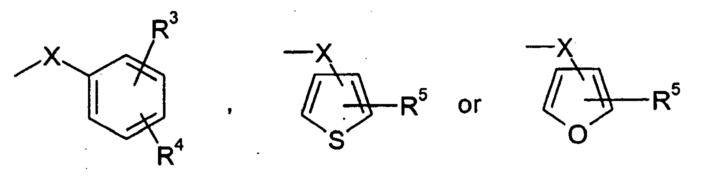 R¹ and R², independently from one another, represent H, linear or branched (C₁-C₄)alkyl, (C₁-C₄)cycloalkyl, (C₁-C₄)cycloalkyl(C₁-C₄)alkyl, halogen or,
together form a group -(CH₂)ₙ-, with n = 1 to 4,
R³ and R⁴, independently from one another, represent H, linear or branched (C₁-C₄)alkyl, OH, linear or branched (C₁-C₄)alkoxy, halogen, CF₃, NO₂, NH₂, NHR' where R' is linear or branched (C₁-C₆)acyl, NHR", N(R")₂, where R" is linear or branched (C₁-C₄)alkyl, optionally substituted anilino, optionally substituted phenoxy, optionally substituted benzyl, or optionally substituted benzoyl,
R⁵ is H, linear or branched (C₁-C₄)alkyl, or halogen,
R⁶ is H or halogen, linear or branched (C₁-C₄)alkyl, (C₁-C₄)cycloalkyl, (C₁-C₄)cycloalkyl(C₁-C₄)alkyl,
X is -CH₂-, -CO-, -O-, -S-, -NH-, or -NR"'- , where R'" is linear or branched (C₁-C₄)alkyl,
and their pharmaceutically acceptable salts,
for the preparation of a pharmaceutical composition useful for the treatment of diabetes, metabolic syndrome, related disorders and obesity.

2. Use according to claim 1 of a compound of formula (I) wherein:
B is CH,
R and R⁶ are each H,
-Y-Z- is 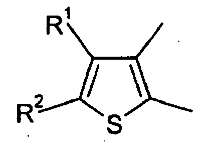
wherein
R¹ is H, methyl, ethyl, isopropyl, isobutyl, cyclopropyl,
R² is H, Cl, Br or methyl,
or,
R¹ and R² together form the group -(CH₂)₄-.

3. Use according to claim 1 or 2 wherein the compound of formula (I) is chosen from among:
- 4-Hydroxy-2,3-dimethyl-5-phenyl-7*H-*thieno[2,3*-b*]pyridin-6-one;
- 2-Ethyl-4-hydroxy-5-phenyl-7*H-*thieno[2,3*-b*]pyridin-6-one;
- 2-Bromo-4-hydroxy-3-methyl-5-phenyl-7*H*-thieno[2,3*-b*]pyridin-6-one;
- 2-Bromo-4-hydroxy-5-phenyl-7*H-*thieno[2,3*-b*]pyridin-6-one;
- 2-Chloro-4-hydroxy-3-isopropyl-5-phenyl-7*H-*thieno[2,3*-b*] pyridin-6-one;
- 3-*sec*-Butyl-2-chloro-4-hydroxy-5-phenyl-7*H*-thieno[2,3-*b*] pyridin-6-one;
- 4-Hydroxy-3-phenyl-5,6,7,8-tetrahydro-1*H*-benzo[4,5]thieno [2,3-*b*]pyridin-2-one; and
- 3-Cyclopropyl-4-hydroxy-5-phenyl-7*H*-thieno[2,3-*b*]pyridin-6-one.

4. Pharmaceutical preparation comprising at least one compound as defined in any of claims 1 to 3, and/or one of its physiologically harmless salts.

5. Pharmaceutical composition according to claim 4, wherein at least said one compound, and/or physiologically harmless salts, is present in an amount ranging from 1 mg to 500 mg, preferably from 5 mg to 100 mg, per dosage unit.

6. Use of a compound of formula (I) according to any of claims 1 to 3, or of one of its pharmaceutically acceptable salts, for the preparation of a medicament.

7. Use of a compound of formula (I) according to any of claims 1 to 3, or of one of its pharmaceutically acceptable salts, for the preparation of a medicament for the treatment of diabetes, metabolic syndrome, related disorders and obesity.

8. Process for producing a pharmaceutical preparation, wherein at least one compound of formula (I), as defined in any of claims 1 to 3, and/or one of its pharmaceutically acceptable salts, is brought into a dosage form together with at least one solid, liquid or semi-liquid carrier and/or auxiliary substance.

9. Compound of formula (I_{A}): 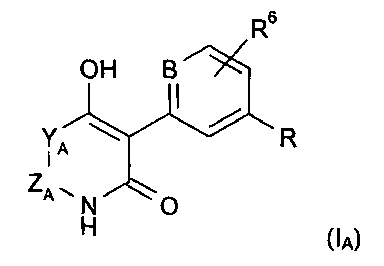 wherein 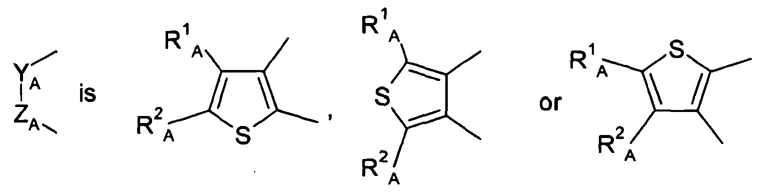 in which R¹_{A} and R²_{A}, independently from one another, represent (C₁-C₄)cycloalkyl or (C₁-C₄)cycloalkyl(C₁-C₄)alkyl or,
together form a group -(CH₂)ₙ-, with n = 1 to 4,
B, R, R³, R⁴, R⁵, R⁶ and X being as defined in claim 1,
its pharmaceutically acceptable salts, as well as stereoisomers and/or diastereoisomers, oxides, solvates and tautomeric forms thereof.

10. Compound of formula (I_{C}): 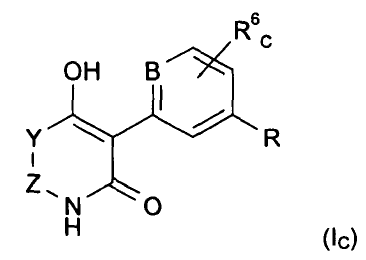 in which
R⁶ is linear or branched (C₁-C₄)alkyl, (C₁-C₄)cycloalkyl, or (C₁-C₄)cycloalkyl(C₁-C₄)alkyl,
B, -Y-Z-, R, R¹, R², R³, R⁴, R⁵ and X being as defined above in claim 1,
its pharmaceutically acceptable salts, as well as stereoisomers and/or diastereoisomers, oxides, solvates and tautomeric forms thereof,
excluding 4-hydroxy-5-(2-methylphenyl)-thieno[2,3-b]pyridine-6(7H)-one.

## Patentansprüche

1. Verwendung einer Verbindung der Formel (I): 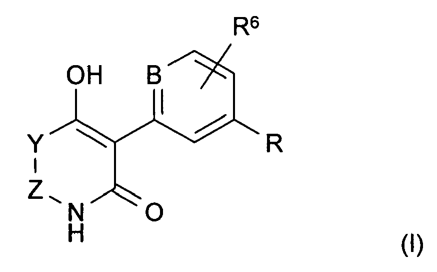 in der
B CH oder N bedeutet, 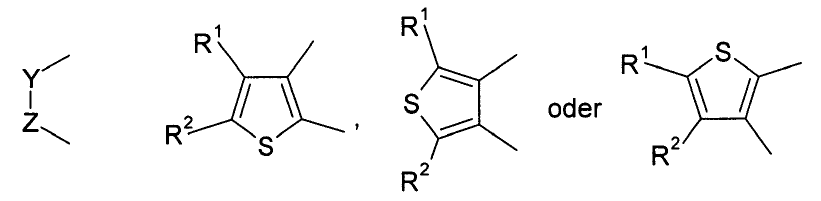 bedeutet,
R H, 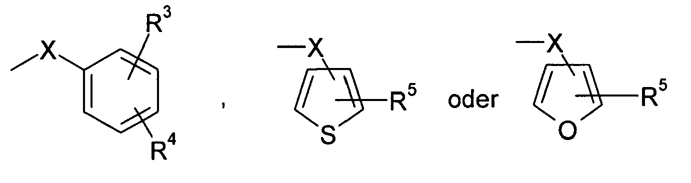 bedeutet
R¹ und R² unabhängig voneinander H, lineares oder verzweigtes (C₁-C₄)Alkyl, (C₁-C₄)Cycloalkyl, (C₁-C₄)Cycloalkyl(C₁-C₄)alkyl, Halogen bedeuten oder
zusammen eine Gruppe -(CH₂)ₙ- bilden, wobei n = 1 bis 4,
R³ und R⁴ unabhängig voneinander H, lineares oder verzweigtes (C₁-C₄)Alkyl, OH, lineares oder verzweigtes (C₁-C₄)Alkoxy, Halogen, CF₃, NO₂, NH₂, NHR', wobei R' lineares oder verzweigtes (C₁-C₆)Acyl bedeutet, NHR", N(R")₂, wobei R" lineares oder verzweigtes (C₁-C₄)Alkyl bedeutet, gegebenenfalls substituiertes Anilino, gegebenenfalls substituiertes Phenoxy, gegebenenfalls substituiertes Benzyl, oder gegebenenfalls substituiertes Benzoyl bedeuten,
R⁵ H, lineares oder verzweigtes (C₁-C₄)Alkyl oder Halogen bedeutet,
R⁶ H oder Halogen, lineares oder verzweigtes (C₁-C₄)Alkyl, (C₁-C₄)-Cycloalkyl, (C₁-C₄)Cycloalkyl(C₁-C₄)alkyl bedeutet,
X -CH₂-, -CO-, -O-, -S-, -NH- oder -NR"'- bedeutet, wobei R'" lineares oder verzweigtes (C₁-C₄)Alkyl bedeutet,
und ihrer pharmazeutisch annehmbaren Salze,
zur Herstellung einer pharmazeutischen Zusammensetzung, die sich für die Behandlung von Diabetes, metabolischem Syndrom, verwandten Erkrankungen und Obesitas eignet.

2. Verwendung nach Anspruch 1 einer Verbindung der Formel (I),
worin:
B CH bedeutet,
R und R⁶ jeweils H bedeuten,
-Y-Z- 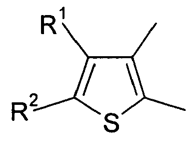 bedeutet,
worin
R¹ H, Methyl, Ethyl, Isopropyl, Isobutyl, Cyclopropyl bedeutet,
R² H, Cl, Br oder Methyl bedeutet
oder
R¹ und R² zusammen die Gruppe -(CH₂)₄- bilden.

3. Verwendung nach Anspruch 1 oder 2, worin die Verbindung der Formel (I) ausgewählt ist aus:
- 4-Hydroxy-2,3-dimethyl-5-phenyl-7*H*-thieno[2,3-*b*]pyridin-6-on;
- 2-Ethyl-4-hydroxy-5-phenyl-7*H*-thieno[2,3-*b*]pyridin-6-on;
- 2-Brom-4-hydroxy-3-methyl-5-phenyl-7*H*-thieno[2,3-*b*]pyridin-6-on;
- 2-Brom-4-hydroxy-5-phenyl-7*H*-thieno[2,3-*b*]pyridin-6-on;
- 2-Chlor-4-hydroxy-3-isopropyl-5-phenyl-7*H*-thieno[2,3-*b*]pyridin-6-on;
- 3-sec-Butyl-2-chlor-4-hydroxy-5-phenyl-7*H*-thieno[2,3-*b*]pyridin-6-on;
- 4-Hydroxy-3-phenyl-5,6,7,8-tetrahydro-1*H*-benzo[4,5]thieno[2,3-*b*]pyridin-2-on und
- 3-Cyclopropyl-4-hydroxy-5-phenyl-7*H*-thieno[2,3-*b*]pyridin-6-on.

4. Pharmazeutische Zubereitung, die mindestens eine Verbindung wie in einem der Ansprüche 1 bis 3 definiert und/oder eines ihrer physiologisch unbedenklichen Salze enthält.

5. Pharmazeutische Zusammensetzung nach Anspruch 4, worin mindestens die eine Verbindung und/oder physiologisch unbedenkliche Salze in einer Menge im Bereich von 1 mg bis 500 mg, vorzugsweise von 5 mg bis 100 mg, pro Dosierungseinheit vorliegt.

6. Verwendung einer Verbindung der Formel (I) nach einem der Ansprüche 1 bis 3 oder eines ihrer pharmazeutisch annehmbaren Salze zur Herstellung eines Medikaments.

7. Verwendung einer Verbindung der Formel (I) nach einem der Ansprüche 1 bis 3 oder eines ihrer pharmazeutisch annehmbaren Salze zur Herstellung eines Medikaments für die Behandlung von Diabetes, metabolischem Syndrom, verwandten Krankheiten und Obesitas.

8. Verfahren zur Herstellung einer pharmazeutischen Zubereitung, worin mindestens eine Verbindung der Formel (I) wie in einem der Ansprüche 1 bis 3 definiert und/oder eines ihrer pharmazeutisch annehmbaren Salze zusammen mit mindestens einem festen, flüssigen oder halbflüssigen Trägerstoff und/oder Hilfsstoff in eine Darreichungsform gebracht wird.

9. Verbindung der Formel (I_{A}): 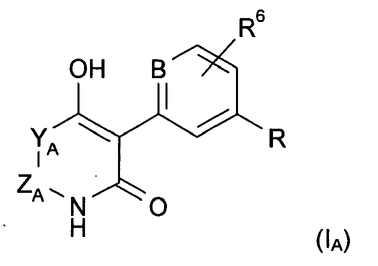 worin 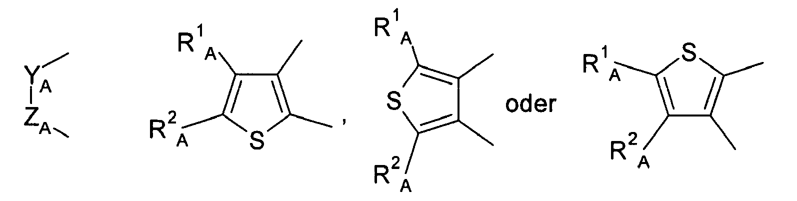 bedeutet,
in denen R¹_{A} und R²_{A} unabhängig voneinander (C₁-C₄)Cycloalkyl oder (C₁-C₄)Cycloalkyl(C₁-C₄)alkyl bedeuten oder
zusammen eine Gruppe -(CH₂)ₙ- bilden, wobei n = 1 bis 4,
wobei B, R, R³, R⁴, R⁵, R⁶ und X wie in Anspruch 1 definiert sind,
ihre pharmazeutisch annehmbaren Salze sowie Stereoisomere und/oder Diastereoisomere, Oxide, Solvate und tautomere Formen davon.

10. Verbindung der Formel (I_{C}): 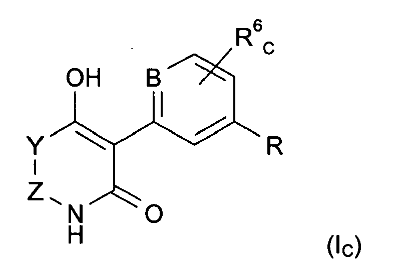 in der
R⁶ lineares oder verzweigtes (C₁-C₄)Alkyl, (C₁-C₄)Cycloalkyl oder (C₁-C₄)Cycloalkyl(C₁-C₄)alkyl bedeutet,
wobei B, -Y-Z-, R, R¹, R², R³, R⁴, R⁵ und X wie in Anspruch 1 oben definiert sind,
ihre pharmazeutisch annehmbaren Salze sowie Stereoisomere und/oder Diastereoisomere, Oxide, Solvate und tautomere Formen davon,
wobei 4-Hydroxy-5-(2-methylphenyl)-thieno[2,3-b]pyridin-6(7H)-on ausgeschlossen ist.

## Revendications

1. Utilisation d'un composé de formule (I) : 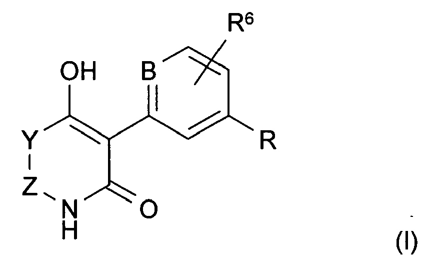 dans laquelle
B est CH ou N, 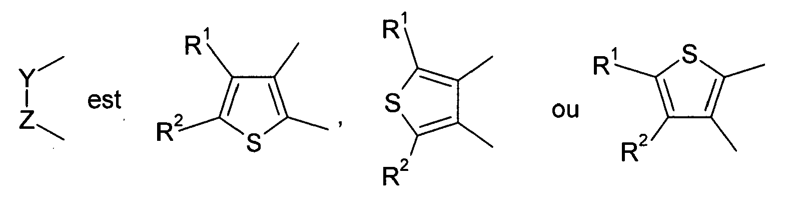 R est H, 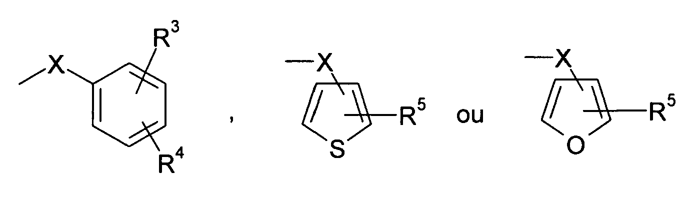 R¹ et R² représentent, indépendamment l'un de l'autre, H, (C₁-C₄)-alkyle, (C₁-C₄)cycloalkyle, (C₁-C₄)cycloalkyl(C₁-C₄)alkyle, linéaire ou ramifié, halogène, ou
forment ensemble un groupement -(CH₂)ₙ-, avec n = 1 à 4,
R³ et R⁴, représentent, indépendamment l'un de l'autre, H, (C₁-C₄)-alkyle linéaire ou ramifié, OH, (C₁-C₄)alcoxy linéaire ou ramifié, halogène, CF₃, NO₂, NH₂, NHR' où R' est (C₁-C₆)acyle linéaire ou ramifié, NHR", N(R")₂, où R" est (C₁-C₄)alkyle linéaire ou ramifié, anilino éventuellement substitué, phénoxy éventuellement substitué, benzyle éventuellement substitué, ou benzoyle éventuellement substitué,
R⁵ est H, (C₁-C₄)alkyle linéaire ou ramifié, ou halogène,
R⁶ est H ou halogène, (C₁-C₄)alkyle, (C₁-C₄)cycloalkyle, (C₁-C₄)cyclo-alkyl(C₁-C₄)alkyle, linéaire ou ramifié,
X est -CH₂-, -CO-, -O-, -S-, -NH-, ou -NR"'-, où R"' est (C₁-C₄)alkyle linéaire ou ramifié,
et ses sels pharmaceutiquement acceptables,
pour la préparation d'une composition pharmaceutique utile pour le traitement du diabète, du syndrome métabolique, des troubles apparentés et de l'obésité.

2. Utilisation selon la revendication 1 d'un composé de formule (I) dans laquelle :
B est CH,
R et R⁶ sont chacun H,
-Y-Z- est 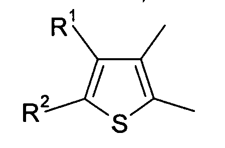
où
R¹ est H, méthyle, éthyle, isopropyle, isobutyle, cyclopropyle,
R² est H, CI, Br ou méthyle, ou
R¹ et R² forment ensemble le groupement -(CH₂)₄-.

3. Utilisation selon la revendication 1 ou 2, dans laquelle le composé de formule (I) est choisi parmi :
- la 4-hydroxy-2,3-diméthyl-5-phényl-7H-thiéno[2,3-b]pyridin-6-one ;
- la 2-éthyl-4-hydroxy-5-phényl-7H-thiéno[2,3-b]pyridin-6-one ;
- la 2-bromo-4-hydroxy-3-méthyl-5-phényl-7H-thiéno[2,3-b]pyri-din-6-one ;
- la 2-bromo-4-hydroxy-5-phényl-7H-thiéno[2,3-b]pyridin-6-one ;
- la 2-chloro-4-hydroxy-3-isopropyl-5-phényl-7H-thiéno[2,3-b] pyridin-6-one ;
- la 3-sec-butyl-2-chloro-4-hydroxy-5-phényl-7H-thiéno[2,3-b] pyridin-6-one ;
- la 4-hydroxy-3-phényl-5,6,7,8-tétrahydro-1H-benzo[4,5]thiéno [2,3-b]pyridin-2-one ; et
- la 3-cyclopropyl-4-hydroxy-5-phényl-7H-thiéno[2,3-b]pyridin-6-one.

4. Préparation pharmaceutique comprenant au moins un composé tel que défini selon l'une quelconque des revendications 1 à 3, et/ou l'un de ses sels physiologiquement inoffensifs.

5. Composition pharmaceutique selon la revendication 4, dans laquelle ledit au moins un composé, et/ou ses sels physiologiquement inoffensifs, est présent selon une quantité allant de 1 mg à 500 mg, préférablement de 5 mg à 100 mg, par unité de dosage.

6. Utilisation d'un composé de formule (I) selon l'une quelconque des revendications 1 à 3, ou de l'un de ses sels pharmaceutiquement acceptables, pour la préparation d'un médicament.

7. Utilisation d'un composé de formule (I) selon l'une quelconque des revendications 1 à 3, ou de l'un de ses sels pharmaceutiquement acceptables, pour la préparation d'un médicament destiné au traitement du diabète, du syndrome métabolique, des troubles apparentés et de l'obésité

8. Procédé de production d'une préparation pharmaceutique, dans laquelle au moins un composé de formule (I), tel que défini selon l'une quelconque des revendications 1 à 3, et/ou l'un de ses sels pharmaceutiquement acceptables, est mis sous une forme de dosage conjointement avec au moins un véhicule et/ou une substance auxiliaire solide, liquide ou semi-liquide.

9. Composé de formule (I_{A}) : 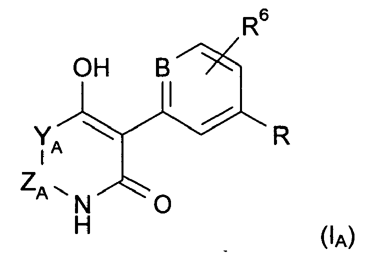 dans laquelle 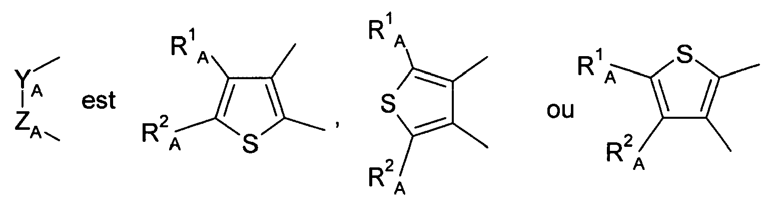 où R¹_{A} et R²_{A}, représentent, indépendamment l'un de l'autre, (C₁-C₄)-cycloalkyle ou (C₁-C₄)cycloalkyl(C₁-C₄)alkyle ou forment ensemble un groupement -(CH₂)ₙ-, avec n = 1 à 4,
B, R, R³, R⁴, R⁵, R⁶ et X étant tels que définis selon la revendication 1, ses sels pharmaceutiquement acceptables, ainsi que les stéréo-isomères et/ou diastéréoisomères, oxydes, solvats et formes tautomères de celui-ci.

10. Composé de formule (I_{C}): 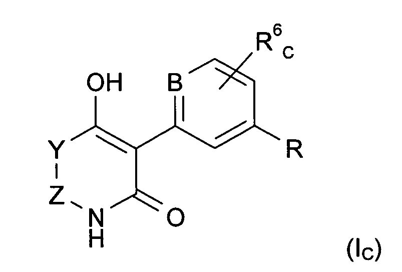 dans laquelle
R⁶ est (C₁-C₄)alkyle, (C₁-C₄)cycloalkyle, (C₁-C₄)cycloalkyl(C₁-C₄)-alkyle, linéaire ou ramifié,
B, -Y-Z-, R, R¹, R², R³, R⁴, R⁵ et X étant tels que définis ci-dessus selon la revendication 1 ,
ses sels pharmaceutiquement acceptables, ainsi que les stéréo-isomères et/ou diastéréoisomères, oxydes, solvats et formes tautomères de celui-ci,
à l'exclusion de la 4-hydroxy-5-(2-méthylphényl)-thiéno[2,3-b]pyridin-6(7H)-one.
